# EUROPÄISCHE PATENTSCHRIFT

(11) **EP 0 413 127 B1**
(45) Veröffentlichungstag und Bekanntmachung des Hinweises auf die Patenterteilung: **20.10.1993**
(21) Anmeldenummer: 90113087.2
(22) Anmeldetag: 09.07.1990
(51) Int. Cl.: A61M 16/16

(54) **Vorrichtung zum Anwärmen und Anfeuchten von Gasen, insbesondere von Atemgasen bei künstlicher Beatmung**
Gas heating and humidification apparatus, especially for respiratory gases used in artificial respiration
Dispositif d'échauffement et d'humidification de gaz, en particulier pour la respiration artificielle

(30) Priorität: 24.07.1989 DE 3924456; 23.01.1990 DE 4001773
(43) Veröffentlichungstag der Anmeldung: 20.02.1991
(73) Patentinhaber: Lang, Volker, Prof. Dr., D-82131 Gauting (DE)
(72) Erfinder: Lang, Volker, Prof. Dr., D-82131 Gauting (DE)
(74) Vertreter: Gossel, Hans K., Dipl.-Ing.

(56) Entgegenhaltungen:
- FR-A- 2 236 519
- FR-A- 2 315 955
- GB-A- 2 001 248
- GB-A- 2 053 695

## Beschreibung

Die Erfindung betrifft eine Vorrichtung zum Anwärmen und Anfeuchten von Gasen, insbesondere von Atemgasen bei der künstlichen Beatmung entsprechend dem Oberbegriff des Hauptanspruchs.

Unter physiologischen Bedingungen dient die Nase der aktiven Anwärmung und Befeuchtung der Atemluft. Beim künstlich beatmeten Patienten jedoch wird diese durch einen Tubus (Schlauch) überbrückt, der mit seinem Ende in der Luftröhre liegt. Die für eine ungestörte Lungenfunktion jedoch unbedingt notwendige Befeuchtung und Erwärmung der Atemgase wird heute durch Vorrichtungen bewerkstelltigt, die vor allem nach zwei unterschiedlichen Prinzipien arbeiten.

Die Vorrichtungen einer ersten Gattung führen der Atemluft aktiv Wärme und Feuchte zu. Dabei wird beispielsweise die trockene und kalte Beatmungsluft durch einen elektrisch beheizten Wasserbad-Anfeuchter geführt und dabei konditioniert, bevor sie dem Patienten zugeführt wird. Die Vorrichtungen der zweiten Gattung arbeiten passiv als Wärme-Feuchte-Austauscher (sog. heat and moisture exchanger, H.M.E.). Hier wird Wärme und Feuchte aus der körperwarmen, feuchten Ausatemluft entzogen, die dann an die kalte, trockene Einatemluft ohne aktive Wärme- und Feuchtezufuhr von außen gegeben wird.

Nach dem heutigen Stand der Technik können zwar die mit einer aktiven Zufuhr von Wärme und Feuchte arbeitenden Vorrichtungen hinreichend gut temperierte und befeuchtete Atemgase liefern, jedoch ist hierzu ein sehr hoher technischer und pflegerischer Aufwand notwendig, so daß die Erstehungs- und Betriebskosten derartiger Geräte sehr hoch sind. Im Gegensatz hierzu ist dieser Aufwand bei den einfach konstruierten und einfach anwendbaren passiven Wärme- und Feuchte-Austauschern nicht veranlaßt. Dafür erbringen diese aber bislang noch keine befriedigende Befeuchtungs- und Anwärmleistung für die künstliche Beatmung.

Eine gattungsgemäße Vorrichtung ist beispielsweise schon aus der GB-A-2053695 bekannt. Hier ist ein Wärme- und Feuchte-Austauscher für Beatmungsgeräte beschrieben, der einen ersten passiven Wärme-Feuchte-Austauscher enthält, der aus einem Bündel hohler Röhrchen besteht, deren Wandungen wasserdurchlässig sind. In einem zweiten Teil des Gehäuses sind ebenfalls Hohlröhrchen angeordnet, deren Wandungen wasserdurchlässig sind und die als aktive Anwärm- und Anfeuchteinrichtung wirken sollen. Um diese Röhrchen wird warmes Wasser geleitet, das Feuchtigkeit und Wärme an die einzuatmende Luft abgeben soll. Zum Feuchtigkeitsaustausch müssen die Wassermoleküle durch die Röhrchenwandungen diffundieren. Hierdurch ergibt sich der Wirkungsgrad der aktiven Anwärm- und Anfeuchteinrichtung.

Ausgehend hiervon stellt sich die Aufgabe, die Funktionsfähigkeit der gattungsgemäßen Vorrichtung zum Anwärmen und Anfeuchten von Gasen zu verbessern.

Diese Aufgabe wird bei einer gattungsgemäßen Vorrichtung durch die zusätzlichen Merkmale des kennzeichnenden Teils des Hauptanspruchs gelöst. Demnach ist der zumindest einen Wasserzuführvorrichtung der aktiven Anwärm- und Anfeuchtvorrichtung eine Saugschicht zugeordnet. Hierdurch ist der Wirkungsgrad der aktiven Anwärm- und Anfeuchtvorrichtung wesentlich verbessert. Bei der erfindungsgemäßen Vorrichtung werden die Vorteile beider eingangs beschriebenen Prinzipien in einer Vorrichtung vereinigt, so daß einerseits eine optimale Befeuchtung und Erwärmung der Atemgase bei gleichzeitig geringem technischem, pflegerischem und finanziellem Aufwand erzielt wird.

In vorteilhafter Weise ist die Vorrichtung derart ausgestaltet, daß sie in einem gegebenenfalls mehrzelligen Gehäuse entlang der Strömungsrichtung des anzufeuchtenden Gases zunächst ein passiver Wärme-Feuchte-Austauscher angeordnet ist, dem eine aktive Anwärm- und Anfeuchtvorrichtung bestehend aus zumindest einer Heizvorrichtung, einer Wasserzuführvorrichtung und einer dieser zugeordneten Saugschicht nachgeschaltet ist, auf die wiederum eine passive Anwärm- und Anfeuchtvorrichtung folgt.

Weitere bevorzugte Ausführungsformen der Erfindung sind in den sich anschließenden Unteransprüchen wiedergegeben.

In Folge wird die Erfindung anhand eines in der Zeichnung dargestellten Ausführungsbeispieles näher erläutert. Es zeigen:

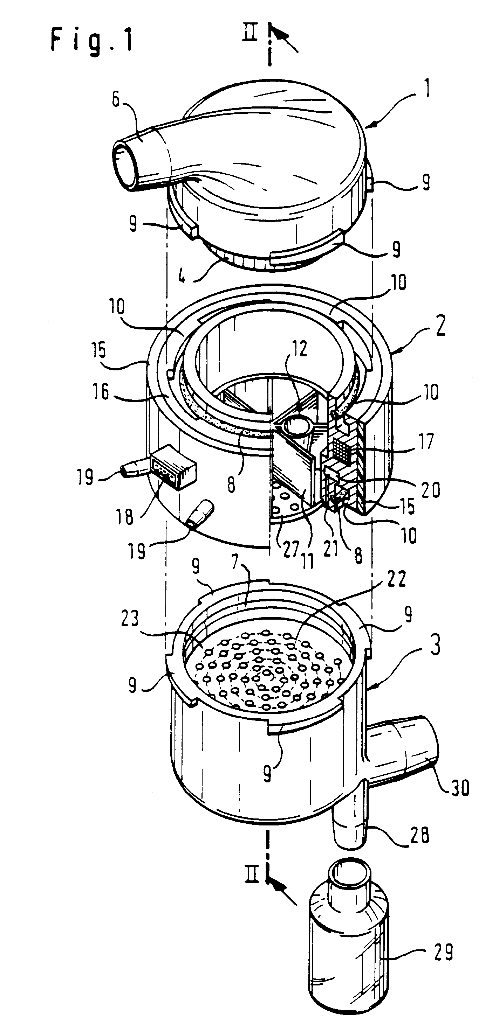

- Fig. 1:: eine perspektivische Explosionsdarstellung eines dreiteilig ausgeführten Ausführungsbeispiels der vorliegenden Erfindung und

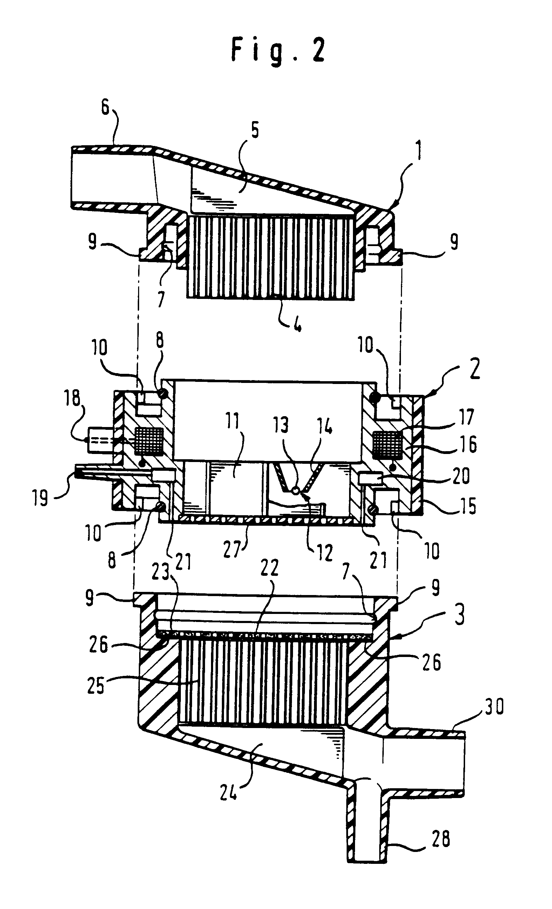

- Fig. 2:: einen Schnitt entlang der Linie II-II durch die Vorrichtung gem. Fig. 1 in zusammengebautem Zustand.

An die als niedriger Kunststoffhohlzylinder ausgebildete durchsichtige Haube 1 der erfindungsgemäßen Vorrichtung ist ein strömungsgünstig geformter Atemgas-Norm-Schlauchstutzen 6 angeformt. In der Haube 1 ist ein erster passiver Wärme-Feuchte-Austauscher 4 derart angeordnet, daß er gegen den gewölbten Gehäusedeckel durch eine dünnwandige Halterippe 5 abgestützt ist. Das durch den Atemgas-Norm-Schlauchstutzen 6 eintretende Gas kann ungehindert über die zahlreichen in der Oberfläche des passiven Wärme-Feuchte-Austauschers 4 mündenden Luftkanälchen in das mittlere Element 2 strömen. Als passiver Wärme-FeuchteAustauscher können handelsübliche Einsätze, wie sie beispielsweise von der Firma Gibeck Respiration A.B., Schweden, vertrieben werden, verwendet werden. Das mittlere Element 2 dient gleichzeitig als mechanisches Verbindungsstück zwischen der Haube 1 und einem Bodenelement 3. Die Verbindung erfolgt über Schnapp-, Dicht- sowie Klemmvorrictungen, beispielsweise Schnapprillen 7, Schnappwülste bzw. O-Ringe 8 bzw. Bajonettverriegelungselemente 9, 10. Innerhalb des mittleren Elements 2 sind Stütz-Wärme-Rippen 11 und ein Strömungsmesser bzw. Strömungssensor im Lumen angeordnet. Der Strömungssensor 12 besteht aus einem geheizten PTC-Widerstand 13 mit vorgeschaltetem Trichter 14. Zur Wärmeisolation ist das mittlere Element 2, welches einen Metallmantel 16 aufweist, mit einem Kunststoffmantel 15 umgeben. Zur thermostatisch kontrollierten Erwärmung des über Stutzen 19 in einen Ringkanal 20 exakt dosiert von einer externen Steuervorrichtung abgegebenen destillierten Sterilwassers ist eine Heizvorrichtung mit zwei NTC-Widerständen 17 eingebettet, die ebenfalls mit der externen Steuervorrichtung, die als solche bereits bekannt ist, über dazugehörige elektrische Anschlüsse 18 verbunden ist. Von diesem Ringkanal 20 wird das Wasser über mehrere Düsen 21 auf die Randzone 23 einer spiralig, siebartig gelochten, dicken, saugfähigen Spezialpapierscheibe 22 abgegeben und durch Kapillarkräfte in dieser gleichmäßig verteilt.

Da die Saugschicht bzw. Spezialpapierscheibe 22 nicht nur auf dem als Kunststoffgehäuse ausgeführten Bodenelement 3 untergebrachten, durch eine dünnwandige Halterippe 24 geschützen zweiten passiven Wärme-Feuchte-Austauscher 25 liegt, sondern zusätzlich mit ihrem Rand 23 an der Unterseite 26 mit dem Kunststoffgehäuse verschweißt ist und von einer im mittleren Element 2 exakt temperierten siebartig gelochten Metallscheibe 27 angedrückt wird, kommt es nach Wassersättigung der Saugschicht 22 zu einer gleichmäßigen, aktiven Befeuchtung und Erwärmung des passiven Wärme-Feuchte-Austauschers 25. Im Überschuß auf diesen zweiten passiven Wärme-Feuchte-Austauscher 25 aufgebrachtes Wasser tropft entsprechend der Schwerkraft über die im passiven Wärme-Feuchte-Austauscher 25 angeordneten Kanälchen ab und trifft auf den geneigt ausgeführten Boden des Bodenelements 3 auf, wo es in den dort am tiefsten Punkt angeordneten Stutzen 28 fließt. Auf den Stutzen 28 ist ein durchsichtiger kleiner Sammelbehälter 29, der auch als Wasserfalle ausgebildet sein kann, aufgesteckt. Damit kann kein im Überschuß auf den passiven Wärme-Feuchte-Austauscher 25 abgegebenes Wasser in den Atemschlauchstutzen 30 und von dort über ein angeschlossenes Adapterteil für den Trachialtubus in die Lunge des Beatmungspatienten gelangen.

In der Folge wird die Funktion des hier anhand des Ausführungsbeispiels erläuterten Erfindungsgegenstandes beschrieben. Trockenes, kaltes Atemgas (ca. 20° Celsius, 10-20 % relative Feuchte) gelangt während der Einatemphase von einer in der Zeichnung nicht dargestellten Beatmungsmaschine über das Patientenschlauchsystem zum Atemgas-Schlauchstutzen der Haube 1. Von hier aus strömt das Atemgas durch den dort eingebauten ersten WärmeFeuchte-Austauscher 4 und wird in einer ersten Stufe durch gespeicherte Wärme und Feuchte passiv vorgewärmt und vorbefeuchtet, wobei diese gespeicherte Wärme und Feuchte zuvor der körperwarmen Ausatemluft des Beatmungspatienten entzogen worden war. Das Atemgas erreicht damit aber nur ca. 30° Celsius bei einer relativen Feuchte von 100 %. Diese Werte sind jedoch noch ungenügend.

In einer zweiten Stufe bewirkt jetzt aber die aktive Befeuchtung und Erwärmung in dem mittleren Element 2 mit der aktiven Anwärm- und Anfeuchtvorrichtung, dem über ein externes Steuergerät exakt dosiert Wasser und elektrische Heizenergie zugeführt wird, daß das durchströmende Atemgas über Wärmerippen 11 zusätzlich weiter erwärmt und befeuchtet wird. Aus dem beheizten Ringkanal 20 und sich daran anschließenden Düsen 21 tritt erwärmtes Wasser aus und fließt über die siebartig gelochte, erwärmte Metallpaltte 27 und von dort in die siebartig gelochte, dicke, saugfähige Spezialpapierscheibe 22, wo sich das Wasser aufgrund der Kapillarwirkung gleichmäßig verteilt. Von der Spezialpapierscheibe 22 aus breitet sich das Wasser gleichmäßig in den vielen Kanälen des zweiten passiven Wärme-Feuchte-Austauschers 25 aus, dessen Oberfläche aufgrund der Vielzahl der Kanäle sehr groß ist. Durch diese Nacherwärmung und Nachbefeuchtung erreicht das Atemgas selbst noch bei großen Atemminutenvolumina inspiratorisch mühelos Temperaturen von 36-37. Celsius. In der Ausatemphase strömt das feuchte, körperwarme Atemgas (36-37° Celsius, 100 % relative Feuchte) über Stutzen 30 durch den zweiten passiven Wärme-Feuchte-Austauscher 25 und nachfolgend durch den ersten passiven Wärme-Feuchte-Austauscher 4 und gibt dabei insgesamt mindestens 70-80 % der Feuchte und Wärme ab, wobei der Großteil dieser Wärme und Feuchte an den ersten passiven Wärme-Feuchte-Austauscher abgegeben wird. Das führt dazu, daß sich in den Ausatemschläuchen nur noch sehr kleine Kondensatmengen bilden. Damit ergibt sich gegenüber konventionellen Systemen zusätzlich ein Vorteil, der sich nicht nur im pflegerischen Aufwand, sondern insbesondere auch in bakteriologischhygienischer Hinsicht zeigt.

Da bei der erfindungsgemäßen Vorrichtung der technisch komplexeste Teil, nämlich das mittlere Element 2, als leicht zu reinigendes, dampfsterilisierbares Mehrmalgebrauch-Verbindungsstück für die zwei preiswert in Einmalgebrauchsausführung steril herstellbaren Wärme-Feuchte-Austauscher 4 und 25 mit den Plastikgehäusen 1 und 3 herstellbar ist, bedeutet dies beim Einsatz nicht nur die optimale Konditionierung und Temperierung bzw. Befeuchtung der Atemgase für den Beatmungspatienten, sondern darüber hinaus auch Schutz vor Infektionen, wobei die Vorrichtung wirtschaftlich eingesetzt werden kann.

Durch den Einbau des Strömungssensors 12, der erst bei Registrierung von Atemhüben der Beatmungsmaschine - d.h. rhytmischer Kühlung des PTC-Widerstandes - die Wasser- und elektische Energiezufuhr aus dem externen Steuergerät freigibt, wird zusätzlich eine hohe Patientensicherheit der erfindungsgemäßen Vorrichtung erreicht, da auch bei extremen Betriebsbedingungen verhindert wird, daß heiße Atemgase oder Wasser in die Trachea des Patienten eindringen.

## Patentansprüche

1. Vorrichtung zum Anwärmen und Anfeuchten von Gasen, insbesondere von Atemgasen bei der künstlichen Beatmung, bestehend aus einer Kombination von mindestens einem passiven Wärme-Feuchte-Austauscher (4,25) und mindestens einer aktiven Anwärm- und Anfeuchtvorrichtung (17-22) die zumindest eine Wasserzuführvorrichtung (19-21) umfaßt,
**dadurch gekennzeichnet,**
daß der zumindest einen Wasserzuführvorrichtung (19-21) der aktiven Anwärm- und Anfeuchtvorrichtung eine Saugschicht (22) zugeordnet ist.

2. Vorrichtung nach Anspruch 1, dadurch gekennzeichnet, daß in einem gegebenenfalls mehrteiligen Gehäuse (1,2,3) entlang der Strömungsrichtung des anzufeuchtenden Gases zunächst ein passiver Wärme-Feuchte-Austauscher (4) angeordnet ist, dem die aktive Anwärm- und Anfeuchtvorrichtung (19-22), die eventuell eine Heizvorrichtung (17) umfaßt, nachgeschaltet ist, auf die wiederum eine passive Anwärm- und Anfeuchtvorrichtung (25) folgt.

3. Vorrichtung nach Anspruch 1 oder 2, dadurch gekennzeichnet, daß ein Strömungssensor (12) integriert ist, der bei Registrierung eines Gasvolumenstromes über eine Steuereinrichtung die aktive Anwärm- und Anfeuchtvorrichtung aktiviert.

4. Vorrichtung nach Anspruch 3, dadurch gekennzeichnet, daß der Strömungsmesser (12) aus einem PTC-Widerstand (13) mit vorgeschaltetem Trichter (14) besteht.

5. Vorrichtung nach einem der Ansprüche 1-4, dadurch gekennzeichnet, daß das Gehäuse dreigeteilt ist, wobei in einer Haube (1) mit Anschlußstutzen (6) der erste passive WärmeFeuchte-Austauscher (4) angeordnet ist, in einem mittleren Element (2) ein Teil der aktiven Anwärm- und Anfeuchtvorrichtung mit Anschlüssen angeordnet ist und im Bodenelement (3) der zweite passive Wärme-Feuchte-Austauscher (4) angeordnet ist, wobei dieser von der Saugschicht (22) zur offenen Seite des Bodenelements (3) hin abgedeckt ist.

6. Vorrichtung nach Anspruch 5, dadurch gekennzeichnet, daß der Boden geneigt ausgebildet ist und daß an dessen tiefsten Punkt ein Wassersammelstutzen (28) mit nachgeschaltetem Sammelbehälter (29) und/oder nachgeschalteter Kühlfalle angeordnet ist.

7. Vorrichtung nach Anspruch 5 oder 6, dadurch gekennzeichnet, daß die Haube (1) und das Bodenteil (3) aus Kunststoff gefertigt sind und mit den passiven Wärme-Feuchte-Austauschern (4,25) und der Saugschicht (22) sterilisierbare und steril verpackbare Baueinheiten bilden.

8. Vorrichtung nach einem der Ansprüche 5-7, dadurch gekennzeichnet, daß das mittlere Element (2) als Verbindungselement für die Haube (1) und das Bodenteil (3) ausgestaltet ist, wobei es vorzugsweise aus Metall gefertigt ist.

9. Vorrichtung nach Anspruch 8, dadurch gekennzeichnet, daß das mittlere Element (2) einen thermostatisierbaren Heizblock mit Wasserkanälen (19,20,21) vorzugsweise zur Erwärmung von Atemgasen, der Anfeuchtflüssigkeit und des im Bodenteil (3) angeordneten Wärme-Feuchte-Austauschers (25) darstellt.

10. Vorrichtung nach einem der Ansprüche 1-9, dadurch gekennzeichnet, daß das mittlere Element (2) oder nur Teile davon und gegebenenfalls ein im Bodenteil (3) angeordneter WärmeFeuchte-Austauscher (25) durch extern eingespeistes thermostatisiertes Wasser über Kanäle oder Rohre erwärmt und befeuchtet wird.

11. Vorrichtung nach einem der Ansprüche 5-10, dadurch gekennzeichnet, daß das mittlere Element (2) mit einem wärmeisolierenden Kunststoffmantel (15) überzogen ist.

12. Vorrichtung nach einem der Ansprüche 2-11, dadurch gekennzeichnet, daß die Saugschicht (22) aus einer siebartig gelochten, dicken, saugfähigen Papierscheibe besteht, die zusammen mit einer exakt temperierten siebartig gelochten Metallscheibe oder einem Metallgitter, von dem sie unter geringem Druck auf den zweiten passiven Wärme-Feuchte-Austauscher (25) gepreßt wird, einen Wärme- und Feuchtigkeitsverteiler bildet.

13. Vorrichtung nach einem der Ansprüche 2-11, dadurch gekennzeichnet, daß die Saugschicht (22) aus einem grobporigen Sintermetall besteht, wobei diese gegebenenfalls mit geringem Druck auf den zweiten passiven Wärme-Feuchte-Austauscher (25) gepreßt wird.

## Claims

1. Gas heating and humidification apparatus, especially for respiratory gases used in artificial respiration, comprising a combination of at least one passive heat and moisture exchanger (4, 25) and at least one active heating and humidification device (17-22) which includes at least one water supply device (19-21),
**characterised in that**
the at least one water supply device (19-21) of the active heating and humidification device is assigned an absorption layer (22).

2. Apparatus according to Claim 1, characterised in that, in a possibly multi-part casing (1, 2, 3) along the direction of flow of the gas to be humidified, initially one passive heat and moisture exchanger (4) is arranged, downstream of which is located the active heating and humidification device (19-22) which possibly contains a heating element (17), this heating and humidification device (19-22) in turn being followed by a downstream passive heating and humidification device (25).

3. Apparatus according to Claim 1 or 2, characterised in that a flow sensor (12) is integrated therein, which on registration of a gas flow rate activates the active heating and humidification device via a control device.

4. Apparatus according to Claim 3, characterised in that the flow sensor (12) consists of a PTC resistor, (13) with a preceding funnel (14).

5. Apparatus according to one of claims 1-4, characterised in that the casing is divided into three, with the first passive heat and moisture exchanger (4) being arranged in a cover (1) having a connection nozzle (6), a part of the active heating and humidification apparatus with connections being arranged in the middle element (2), and the second passive heat and moisture exchanger (4) being arranged in the bottom element (3), the heat and moisture exchanger (4) here being covered by an absorption layer (22) facing the open end of said bottom element (3).

6. Apparatus according to Claim 5, characterised in that the bottom is inclined, and that at its lowest point features a water collection nozzle (28) with downstream collection container (29) and/or a downstream cooling trap.

7. Apparatus according to Claim 5 or 6, characterised in that the cover (1) and the bottom section (3) are manufactured in plastic and form constructional units with the passive heat and moisture exchangers (4, 25) and the absorption layer (22), which constructional units can be sterilised and sterile-packed.

8. Apparatus according to one of claims 5-7, characterised in that the middle element (2) is designed as a connecting element for the cover (1) and the bottom element (3), and is preferably manufactured in metal.

9. Apparatus according to Claim 8, characterised in that the middle element (2) constitutes a thermostatically controllable heater block with water ducts (19, 20, 21) preferably for the heating of respiratory gases, the humidifying liquid and the heat and moisture exchanger (25) arranged in the bottom element (3).

10. Apparatus according to one of claims 1-9, characterised in that the middle element (2), or only parts thereof, and possibly a heat and moisture exchanger (25) arranged in the bottom element (3) are heated and humidified via ducts or tubes by externally supplied thermostatically controlled water.

11. Apparatus according to one of claims 5-10, characterised in that the middle element (2) is covered by a heat-insulating plastic sheath (15).

12. Apparatus according to one of claims 2-11, characterised in that the absorption layer (22) consists of a sieve-like perforated, thick, absorbent paper disk which, together with a precisely conditioned sieve-like perforated metal disk, or a metal grid, by which said absorption layer (22) is pressed with a small degree of pressure onto the second passive heat and moisture exchanger, forms a heat and moisture distributor.

13. Apparatus according to one of claims 2-11, characterised in that the absorption layer (22) consists of a coarsely porous sintered metal, this possibly being pressed with a small degree of pressure onto the second passive heat and moisture exchanger (25).

## Revendications

1. Dispositif d'échauffement et d'humidification de gaz en particulier pour la respiration artificielle, consistant en une combinaison d'au moins un échangeur passif de chaleur/ humidité (4, 25) et d'un dispositif actif d'échauffement et d'humidification (17-22) qui comprend au moins un dispositif d'alimentation en eau (19-21),
caractérisé
en ce qu'à cet au moins unique dispositif d'alimentation en eau (19-21) du dispositif actif d'échauffement et d'humidification, une couche d'aspiration (22) est associée.

2. Dispositif selon la revendication 1, caractérisé en ce que, dans un boîtier (1, 2, 3) le cas échéant en plusieurs parties, un échangeur de chaleur/humidité (4) passif est d'abord disposé le long de la direction du flux du gaz à humidifier, suivi en aval du dispositif d'échauffement et d'humidification (19-22) qui comprend le cas échéant un dispositif de réchauffement, suivi à son tour d'un dispositif passif d'échauffement et d'humidification (25).

3. Dispositif selon les revendications 1 ou 2, caractérisé en qu'un capteur de flux (12) est intégré qui, en enregistrant un débit de gaz active, par l'intermédiaire d'un dispositif de commande, le dispositif d'échauffement et d'humidification.

4. Dispositif selon la revendication 3, caractérisé en ce que le débit-mètre (12) consiste en une résistance PTC (13) avec un entonnoir (14) en amont.

5. Dispositif selon l'une des revendications 1 à 4, caractérisé en ce que le boîtier est en trois parties, dans la première calotte (1) avec tubulures de raccordement (6) étant disposé le premier échangeur passif de chaleur/humidité (4), dans un élément central (2) étant disposée une partie du dispositif actif d'échauffement et d'humidification avec des raccordements et dans l'élément de fond (3), le deuxième échangeur passif de chaleur/humidité (4), celui-ci étant recouvert par la couche d'aspiration (22) en direction du côté ouvert de l'élément de fond (3).

6. Dispositif selon la revendication (5) caractérisé en ce que le fond est incliné et qu'à son point le plus bas une tubulure d'accumulation d'eau (28) avec récipient d'accumulation en aval (29) et/ou une trappe réfrigérante en aval sont disposées.

7. Dispositif selon les revendications 5 ou 6, caractérisé en ce que la calotte (1) et la partie du fond (3) sont fabriquées en plastique et forment avec les échangeurs passifs de chaleur et d'humité (4, 25) et la couche d'aspiration (22) des unités stérilisables et pouvant être emballées de manière stérile.

8. Dispositif selon l'une des revendications 5 à 7, caractérisé en ce que l'élément central (2) est formé comme élément de raccordement pour la calotte (1) et la partie de fond (3), cet élément étant de préférence fabriqué en métal.

9. Dispositif selon la revendication 8, caractérisé en ce que l'élément central (2) représente un bloc de chauffage thermostatisable avec des canalisations d'eau (19, 20, 21) de préférence pour l'échauffement de gaz de respiration, du liquide d'humidification et de l'échangeur de chaleur/humidité (25) disposé dans la partie du fond (3).

10. Dispositif selon l'une des revendications 1 à 9, caractérisé en ce que l'élément central (2), ou même seulement des parties de celui-ci et le cas échéant un échangeur de chaleur/humidité (25) disposé dans la partie du fond (3), est réchauffé et humidifié par des canaux ou tuyaux alimentés de l'extérieur en eau thermostatisable.

11. Dispositif selon l'une des revendications 5 à 10, caractérisé en ce que l'élément central (2) est revêtu d'une couche en plastique thermo-isolante (15).

12. Dispositif selon l'une des revendications 2 à 11, caractérisé en ce que la couche d'aspiration (22) consiste en un disque de papier à la manière d'un tamis troué, épais, absorbant, qui avec un disque métallique ou un grillage métallique troué à la manière d'un tamis et tempéré avec précision est pressé à une faible pression sur le deuxième échangeur passif de chaleur/humidité (25), formant un distributeur de chaleur et d'humidité.

13. Dispositif selon l'une des revendications 2 à 11, caractérisé en ce que la couche d'aspiration (22) consiste en un métal fritté à pores grossiers, celle-ci étant pressée le cas échéant à faible pression sur le deuxième échangeur passif de chaleur/humidité (25).
